# EUROPEAN PATENT SPECIFICATION

(11) **EP 2 934 647 B1**
(45) Date of publication and mention of the grant of the patent: **12.02.2020**
(21) Application number: 13863790.5
(22) Date of filing: 19.12.2013
(51) Int. Cl.: A61M 25/00

(54) **DISTAL CATHETER TIPS AND FORMATION THEREOF**
DISTALKATHETERSPITZEN UND FORMUNG DAVON
EXTRÉMITÉS DISTALES DE CATHÉTER ET LEUR FORMATION

(30) Priority: 21.12.2012 US 201261745341 P
(43) Date of publication of application: 28.10.2015
(73) Proprietor: Volcano Corporation, San Diego, CA 92130 (US)
(72) Inventor: Leblance, Christopher, San Diego, CA 92130 (US); Stigall, Jeremy, San Diego, CA 92130 (US); Sasamine, Kazuo, San Diego, CA 92130 (US)
(74) Representative: de Haan, Poul Erik
(86) International application number: PCT/US2013/076502
(87) International publication number: WO 2014/100392

(56) References cited:
- WO-A2-03/037419
- US-A- 5 403 292
- US-A1- 2005 131 445
- US-A1- 2008 262 470
- US-A1- 2008 262 470
- US-A1- 2009 299 284

## Description

### RELATED APPLICATION

This application claims the benefit of and priority to U.S. Provisional Serial No. 61/745,341, filed December 21, 2012.

### TECHNICAL FIELD

The present disclosure relates generally to catheters for navigating through the human vasculature, and in particular, to improved distal tips for catheters and methods of forming distal tips for catheters.

### BACKGROUND

Catheters such as intravascular catheters are well known for use in diagnostic and therapeutic applications wherein it is necessary to administer a fluid to, or otherwise contact, a precise location within the cardiovascular system, for example, by guiding the tip or distal end of the catheter through branching blood vessels. Such guiding is accomplished in part by manipulation of a proximal portion of the catheter in order to impart forces needed to curve and guide the catheter through the curving and branching blood vessels.

Generally, distal tips of catheters are made by hand. For example, an operator bonds or necks heated material over a mandrel, cools the material, and trims the material to the desired length. If the material is necked incorrectly, the operator has to reheat the part until the correct shape is achieved. The process takes both time and skill.

In addition, consistency of necking between two different operators is difficult to achieve. One operator may neck harder or heat longer than the other. Moreover, the heat being applied may not be transferred consistently between the part and heat torch so that one portion of the part will endure more or less heat.

Beyond manufacturing and consistency problems, prior art catheter tips fail to provide a smooth transition from a more rigid distal shaft of a catheter body. These catheter tips are made of a uniform flexible material and are joined directly to the rigid distal shaft. The abrupt transition between the flexible tip material and the rigid distal shaft risks joint failure and may result in dislocation of the catheter tip. Document WO 03/037419 A2 discloses a catheter tip according to the preamble of claim1.

Therefore, a need exists for improved distal tips and methods of making those distal tips for catheters that reduce human error and cost, and increase reproducibility.

### SUMMARY

The present invention relates to a catheter tip as defined by the features of claim 1 and to a method of making the catheter tip of claim 1, as defined by the features of claim 11. Preferred embodiments of the invention are defined by the appended dependent claims.

In an exemplary embodiment, the method includes providing a mandrel and a holding hypotube. A tip first material, tip second material, and the holding hypotube are assembled over the mandrel. Specifically, the first material is placed over the mandrel and the hypotube, and the second material is placed over the mandrel and under the first material. The first material has an outer diameter than is greater than the outer diameter of the second material. A shrink tube of heat-shrink material is then placed around at least a junction of the first and second materials. The shrink tube is heated, the first and second materials cooled, and the shrink tube and hypotube removed. In some embodiments, the shrink tube, first material, and second material are centered between two heating dies configured to form a circle around the shrink tube, first material, and second material. In one embodiment, the shrink tube, first material, and second material are heated to a temperature of about 121 °C (250 °F) to 260 °C (500 °F). The heating time may be between about 0.25 to 60 seconds. The methods reduce the possibility of human error and increase consistency of the distal tips produced.

In other embodiments, the methods include placing a first polyether block amide having a first Shore D durometer hardness over the mandrel and the hypotube, and placing a second polyether block amide having a second Shore D hardness that is greater than the first Shore D hardness over the mandrel and under the first polyether block amide. The first polyether block amide generally has a Shore D hardness of about 50 to 60, while the second polyether block amide typically has a Shore D hardness of about 60 to 70. In an exemplary embodiment, the first polyether block amide has Shore D hardness of about 55, and the second polyether block amide has a Shore D hardness of about 63.

In alternative embodiments, the methods include providing a holding hypotube with a distal leg and a distal back. The tip first material is placed over the mandrel and the distal leg, and the tip second material is placed over the mandrel and under the first material so that the second material abuts the distal leg. The first material has an outer diameter than is greater than the outer diameter of the second material. In one embodiment, the first material abuts the distal back of the hypotube. In a further aspect, a catheter is formed including a distal tip formed according to the methods described herein.

The midsection includes at least two tip materials in an overlapping configuration and the distal segment includes one of the at least two tip materials. According top the invention, the two tip materials in the overlapping configuration are fused together using the method of the invention. The midsection with the at least two overlapping tip materials and the distal segment with one of the at least two materials delivers a needed transition between a stiff proximal segment of the catheter tip and a relatively flexible distal segment of the catheter tip.

Both the foregoing general description and the following detailed description are exemplary and explanatory in nature and are intended to provide an understanding of the present disclosure without limiting the scope of the present disclosure. In that regard, additional aspects, features, and advantages of the present disclosure will become apparent to one skilled in the art from the following detailed description. References to embodiments throughout the description which are not under the scope of the appended claims merely represent possible exemplary executions and are therefore not part of the present invention.

### BRIEF DESCRIPTIONS OF THE DRAWINGS

Aspects of the present disclosure are best understood from the following detailed description when read with the accompanying figures. It is emphasized that, in accordance with the standard practice in the industry, various features are not drawn to scale. In fact, the dimensions of the various features may be arbitrarily increased or reduced for clarity of discussion. In addition, the present disclosure may repeat reference numerals and/or letters in the various examples. This repetition is for the purpose of simplicity and clarity and does not in itself dictate a relationship between the various embodiments and/or configurations discussed.
FIG. 1 illustrates a subassembly of two tip materials to be bonded according to various aspects of the present disclosure.
FIGS. 2A and 2B are perspective views of the tip first and second materials, respectively, prior to being bonded.
FIG. 3 is a perspective view of a holding hypotube.
FIG. 4 is a diagrammatic cross-section of the subassembly of FIG. 1 taken along line 4-4.
FIG. 5 is a diagrammatic cross-sectional side view of the subassembly of FIG. 1.
FIG. 6 illustrates a tapered distal tip formed according to various examples.
FIG. 7 illustrates a cross-sectional side view of the tapered distal tip coupled to a catheter shaft as shown in FIG. 6.
FIG. 8 illustrates a ridged distal leg of the forming hypotube according to certain examples.
FIG. 9 illustrates a tapered distal tip with a variable stiffness element.

### DETAILED DESCRIPTION

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

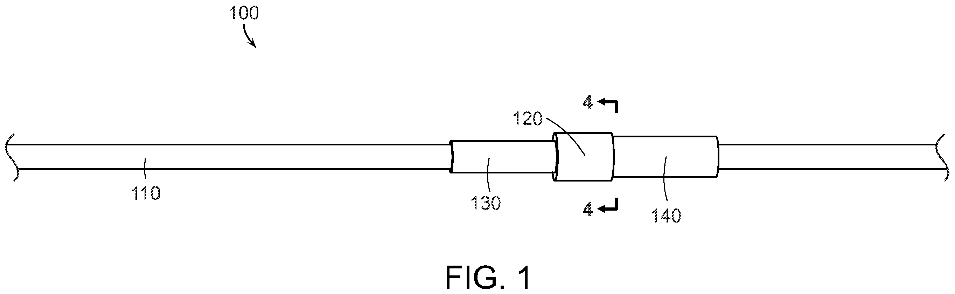

Referring to FIG. 1, a subassembly 100 for forming a tapered distal tip for a catheter is shown. The tip first material 120, tip second material 130 and holding hypotube 140 are assembled over the mandrel 110. The mandrel 110 may be a metal tube or other suitable material thin enough to pass through the inner lumens of tip first material 120, tip second material 130, and holding hypotube 140. The mandrel 110 is positioned in the inner lumens of the tip first material 120 and tip second material 130 to keep the inner lumens open during the fusing of the tip first and second materials 120, 130. In an exemplary embodiment, the mandrel 110 has a diameter of about 1.1 mm (0.042 inches).

The tip first material 120 and tip second material 130 are any materials suitable for forming a flexible distal tip. In an exemplary embodiment, the tip first and second materials 120, 130 include a polyether block amide, such as Pebax® thermoplastic polymers available from Arkema Inc. The flexible materials are inexpensive and create a strong bonding surface that aids in tensile strength. The flexible materials also allow the original shape to be retained after going around tortuous paths. Advantageously, the materials can be used on the distal section of a catheter to guide the unit during an operation.

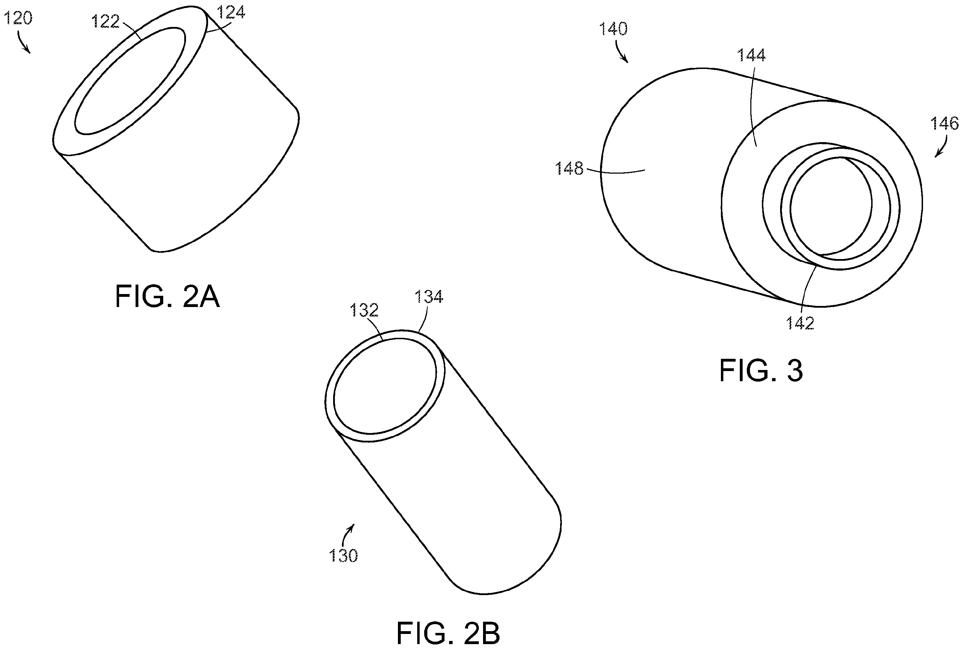

Moving now to FIG. 2A, the tip first material 120 has an inner diameter 122 and an outer diameter 124. In one embodiment, the inner diameter 122 measures about 1.6 mm (0.062 inches) and the outer diameter 124 measures about 2.5 mm (0.100 inches). In another embodiment, the tip first material 120 includes a polyether block amide having a Shore D durometer hardness of about 50 to 60. For example, the tip first material 120 may include Pebax® 55D.

Turning to FIG. 2B, the tip second material 130 has an inner diameter 132 and an outer diameter 134. In one embodiment, the inner diameter 132 measures about 1.3 mm (0.051 inches) and outer diameter 134 measures about 1.6 mm (0.061 inches). In another embodiment, the tip second material 130 includes a polyether block amide having a Shore D durometer hardness of about 60 to 70. For example, the tip second material 130 may include Pebax® 63D.

The outer diameter 124 of the tip first material 120 is greater than the outer diameter 134 of the tip second material 130. This facilitates the method of the present disclosure by allowing tip second material 130 to slide under or within the tip first material 120.

FIG. 3 illustrates a holding hypotube 140. The holding hypotube 140 includes a proximal portion 148 and a distal portion 146. The holding hypotube 140 is a metal alloy tubing that provides support during the manufacturing method. The distal portion 146 includes a distal leg 142 and a distal back 144. The distal leg 142 protrudes from the distal back 144 in one direction and extends into an inner lumen of the proximal portion 148 in another direction. In an exemplary embodiment, the distal leg 142 is a cylindrical projection with an outer diameter of about 1.5 mm (0.059 inches) and an inner diameter of about 1.3 mm (0.050 inches). In one embodiment, the distal leg 142 extends about 0.044 inches from the distal back 144. In an alternative embodiment, the distal back 144 is a shoulder extending between an outer diameter of about 2.5 mm (0.100 inches) for proximal portion 148 and 1.5 mm (0.059 inches) for the outer diameter of the cylindrical projection.

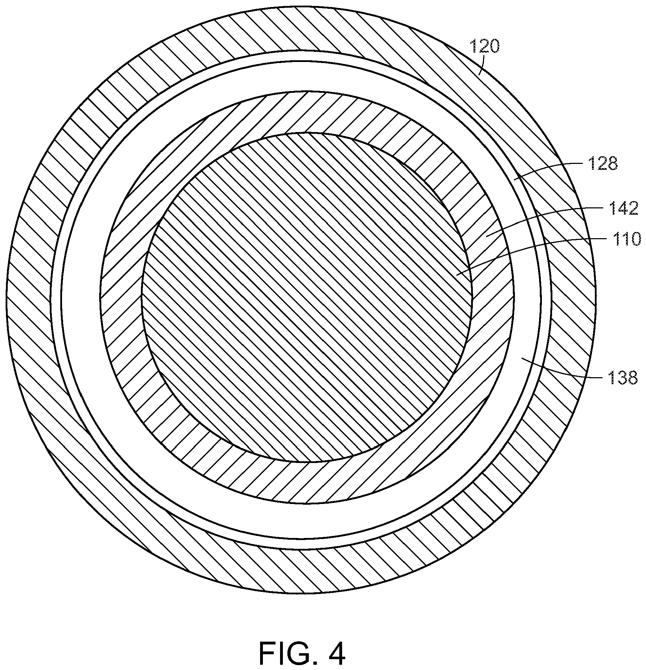

FIG. 4 illustrates the cross-section of the subassembly 100 of FIG. 1 taken along line 4-4. As can be seen, the tip first material 120 is placed over the distal leg 142, and the distal leg 142 is placed over the mandrel 110. The dimensions of the tip first material 120, tip second material 130, the distal leg 142, the distal back 144, and the mandrel 110 are chosen so that this arrangement occurs. The proximal end 138 of the tip second material 120 is shown as its outer diameter is larger than the outer diameter of the cylindrical projection. Between the first tip material 120 and the tip second material 130 is an air gap 128. In an exemplary embodiment, the air gap 128 separates the second tip material 130 and the tip first material 120 by 0.02 mm (0.001 inches). The air gap 128 eases assembly of the components and is eliminated as the parts melt during the manufacturing process.

The method of forming a tapered distal tip will now be described. The method begins by providing the mandrel 110 and holding hypotube 140. The tip first material 120 and the tip second material 130 are cut and placed distally over the mandrel 110 and the holding hypotube 140.

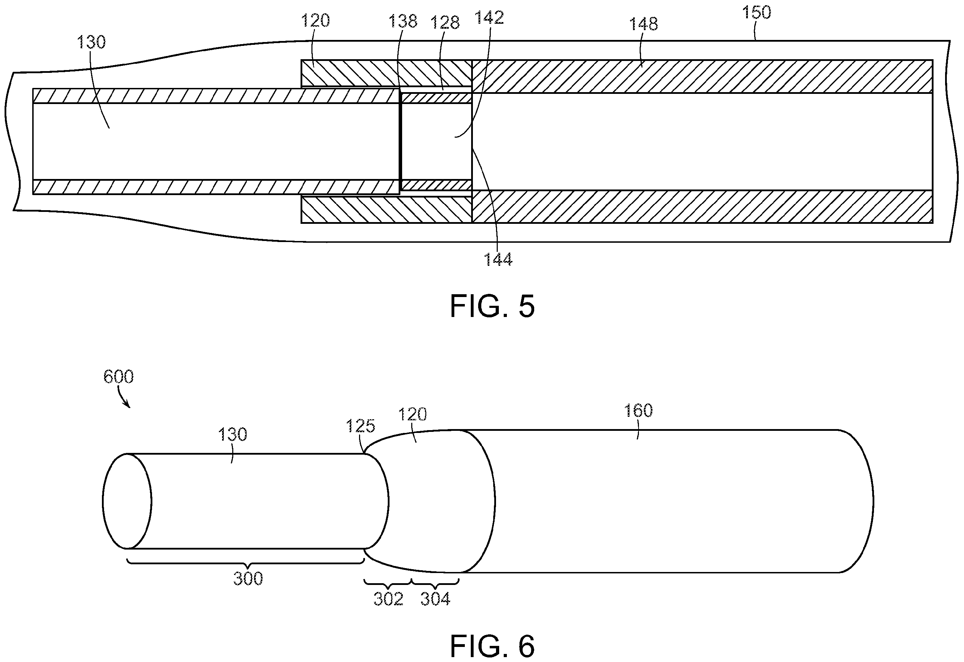

Specifically, referring to FIG. 5, the tip first material 120 is placed over the mandrel and the distal leg 142 of the holding hypotube 140. The tip first material 120 butts up to the distal back 144. The tip first material 120 merely touches the edge of the distal back 144, but does not go over the proximal portion 148 of the holding hypotube 140. The air gap 128 includes the space between the distal leg 142 and the tip first material 120. The tip second material 130 is placed over the mandrel and within the inner lumen of the tip first material 120. The tip second material 130 butts up to the distal leg 142 of the hypotube 140. Since the outer diameter of the distal leg 142 is greater than the inner diameter of the tip second material 130, the proximal end 138 of the tip second material 130 merely touches the edge of the distal leg 142, but does not go over it. The mandrel 110 is removed from FIG. 5 for ease of illustration.

Next, a shrink tube 150 of heat-shrink material is placed over the junction between the tip first material 120 and the tip second material 130, as well as over the holding hypotube 140. Bonding of the tip first and second materials 120,130 is completed by applying heat to the shrink tube 150 to melt the first and second materials 120, 130, while also shrinking the shrink tube 150.

The shrink tube 150 may be manufactured from a material that will prevent a permanent adhesion of the shrink tube 150 to the first and second tip materials 120, 130, so that shrink tube 150 can be easily removed (for example, by peeling off) at the end of the bonding process. Similarly, mandrel 110 may be manufactured from or coated with a material that will not adhere to the inner lumen of the first and second tip materials 120, 130.

In one embodiment, heating shrink tube 150 involves centering shrink tube 150, tip first material 120, and tip second material 130 between two heating dies configured to form a circle around the shrink tube 150, tip first material 120, and tip second material 130. The top of the dies may be used to pre-shrink the shrink tube 150. The shrink tube 150, tip first material 120, and tip second material 130 are heated to between about 121 °C (250 °F) to 260 °C (500 °F) for about 0.25 to 60 seconds. Heat is applied by a hot box and verified with thermocouples.

In an exemplary embodiment, the time and temperature of the heating machine is automatically controlled so the operator's task is limited to pre-shrinking and placement in the dies. Since the placement in the machine may be controlled by a micrometer, the operator is able to place the shrink tube 150, tip first material 120, and tip second material 130 in the same location every time. Any operator can be trained on these steps, increasing the consistency of the formed tip. After placement between the dies, a button may be pushed that triggers the machine to heat for a specific time. Once the appropriate time and temperature are reached, the dies that are heating the shrink tube 150, tip first material 120, and tip second material 130 can automatically open. The operator then cools the part and removes the shrink tube 150.

During heating, the shrink tube 150 shrinks and constrains a flow of first and second materials 120, 130. As first and second materials 120, 130 melt, they fuse together to form a composite tip having different thicknesses and material properties. The distal most portion of the tip is formed entirely of tip second material 130, making it the most flexible area. The proximal portion of the tip is formed entirely of tip first material 120, making it more rigid than the distal portion of tip first material 120. The tapered transition zone 125 is formed of both materials and allows a smooth transition in stiffness between the distal and proximal portions. After cooling first and second materials 120, 130, shrink tube 150 and holding hypotube 140 are removed to yield a flexible distal tip. The inner diameter of the distal tip has been molded during the heating process to match the mandrel 110 outer diameter over most of the length with an enlarged inner diameter matching the outer diameter of the distal leg 142 at the proximal portion.

Referring to FIG. 6, distal tip 600 includes a proximal segment 304, a distal segment 300, and a midsection 302 extending there between. The proximal segment is formed from the first tip material 120 and the distal segment 300 is formed from the second tip material 130. The midsection 302 is formed from both the first tip material 120 and the second tip material 130. The midsection 302 is formed from heat fusing the overlapping configuration of the first tip material 120 and the second tip material 130 (See FIG. 5 and accompanying text). Shrink tube 150 created a smooth and long transition zone from the tip first material 120 to the tip second material 130 over their junction. The distal tip 600 tapers from the proximal segment 304 to the distal segment 300. The resulting bond is strong and flexible, with a transition zone of blended material properties, rather than an abrupt transition. Alternatively, the overlapping section can be formed by an adhesive bond between the first tip material 120 and a second tip material 130. In an exemplary embodiment, the length of the distal tip 600 is about 12 mm.

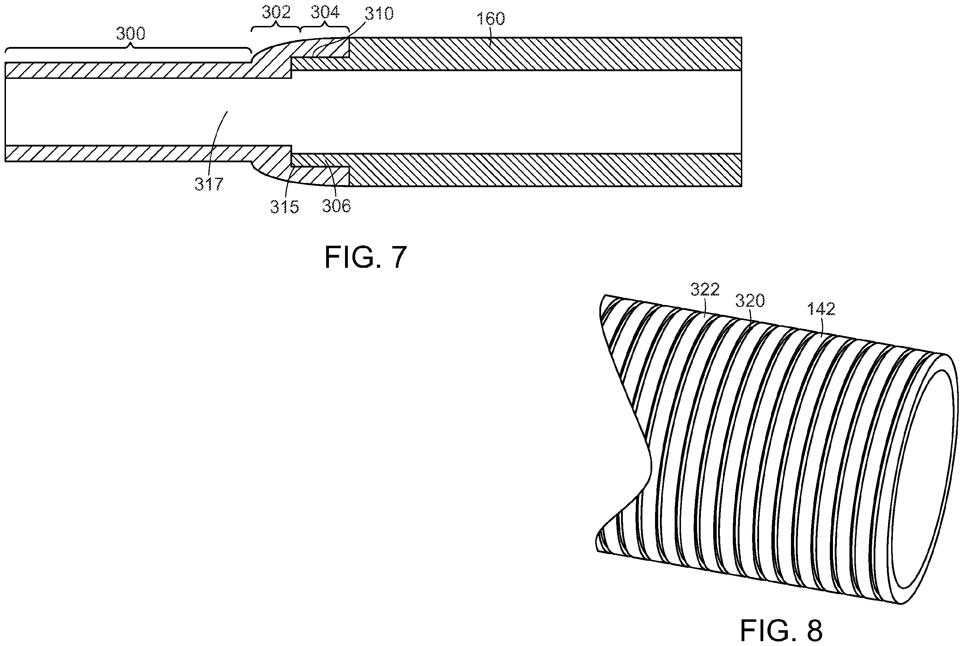

The proximal segment 304 of the distal tip 600 is configured to operably couple to a shaft 160 of a catheter body. Typically, the shaft 160 is a hypotube. In certain embodiments, the proximal segment 304 is configured to operably couple to an imaging hypotube of a catheter body. The proximal segment 340 can couple to the catheter shaft 160 using any bonding technique and any joint known in the art, for example, lap joints or butt joints. Preferably, the catheter shaft 160 and the proximal segment 304 are joined together in a lap joint configuration. FIG. 7 illustrates a lap joint configuration for coupling the distal tip 600 and the catheter shaft 160. For a lap joint configuration, the catheter shaft 160 includes a distal extension 306 having a diameter smaller than an inner diameter 122 of the proximal segment 304. As a result, the distal extension 305 is able to closely fit into a lumen 317 of the proximal segment 304. The distal tip 600 is fused to the catheter shaft 160, for example, using a shrink tube and applying heat to the joint. As an alternative to or in combination with heat fusion, the distal extension 306 is coupled to the proximal segment in the lap joint configuration using an adhesive.

In certain embodiments, an inner surface 310 of the proximal segment 304 or an outer surface 315 of the distal extension 306 include a surface modification to increase the bond strength between the two. In one embodiment, an outer surface 315 of the distal extension 306 or an inner surface 310 of the proximal segment 304 may include one or more ridges to increase the strength of the lap joint. In order to create one or more ridges on the inner surface 310 of the proximal segment, 304, the proximal leg 142 of the holding hypotube 140 includes one or more ridges 322 (as shown in FIG. 8). The one or more ridges 322 on the proximal leg 142 can be formed by adding notches or indentations on the proximal leg 142 manually, chemically, electrically, by machinery, water cutting, ect. During heat fusion of the first tip material 120 and the second tip material 130, the inner surface 310 of the first tip material 120 (proximal segment 304) conforms to the proximal leg 142 of the holding hypotube 140 (See FIG. 5). As the first tip material 120 conforms to the proximal leg 142, ridges form on the inner surface 310 as the first tip material 120 expands into the indentations 320 of the ridges 322 of the proximal leg 142.

As discussed, the outer surface 315 of the distal extension 306 can also include one or more ridges to create an enhanced binding surface. The one or more ridges on the distal extension 306 can be formed by adding notches or indentations on the outer surface manually, chemically, electrically, by machinery, water cutting, etc.

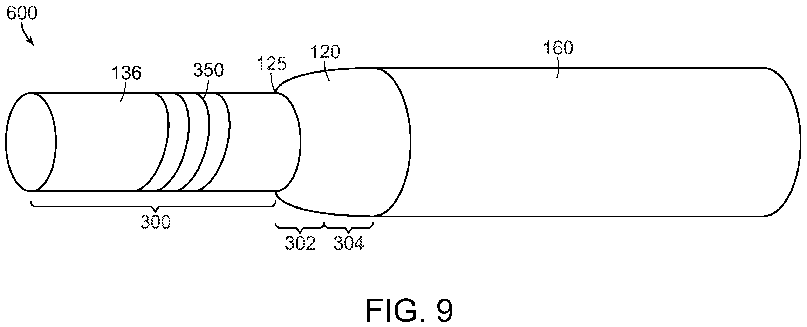

In certain embodiments, the proximal segment 304, the distal segment 300, and/or the midsection of the distal tip 600 include a variable stiffness element. The variable stiffness element is designed to increase the flexibility of one or more segments of the distal tip 600. In one embodiment, the variable stiffness element is a spiral cut pattern or notched pattern cut into one or more segments of the distal tip, which will provide flexibility to that segment. A spiral cut pattern 350 includes cutting out a portion of material of the formed distal tip 600 in a spiral pattern, as exemplified in FIG. 9. For a notch-cut pattern, a portion of material of the formed distal tip 600 is cut out in a non-spiral pattern. Although FIG. 9 illustrates a distal segment 300 of the distal tip 600 with a spiral cut pattern 350, it is understood that any combination of segments of the distal tip 600 can include a spiral cut or notch cut pattern.

The methods described herein are simpler, less expensive, save time, reduce the possibility of human error and improve reproducibility by introducing automated machines. Automated heating devices help control the consistency of the tip, which lowers the scrap ratio. There is no added step for a mis-shaped part, reducing the assembly time. This process can be duplicated by any operator, giving a more lean manufacturing line and improving the consistency.

The tip can be made as a sub-assembly, which increases stock and ultimately saves time and money. Also, time and money decrease because the two materials can be ordered in bulk and pre-trimmed.

The distal tip and methods for forming the distal tip of the invention are applicable to any intraluminal device such as guidewires and catheters. The guidewires and catheters with the inventive distal tip can be imaging device, interventional device, and combinations thereof. The imaging device may incorporate ultrasound technology, photoacoustic technology, optical coherence tomography technology, etc. The interventional device may be configured to perform ablations, aspiration, morcellation, etc.

## Claims

1. A catheter tip (600) comprising a proximal segment (304), a distal segment (300), and a midsection (302) there between, wherein the midsection includes at least two tip materials (120, 130) in an overlapping configuration and fused together and the distal segment includes one of the at least two tip materials, **characterized in that** the midsection provides a transition in stiffness between the proximal segment and the distal segment, wherein the proximal segment is stiffer than the distal segment.

2. The catheter tip of claim 1, wherein the catheter tip tapers from the proximal segment to the distal segment.

3. The catheter tip of claim 1, wherein the proximal segment is configured to operably couple to a shaft of a catheter body and to overlap with a portion of the shaft of the catheter body.

4. The catheter tip of claim 1, wherein the at least two tip materials include a first tip material and a second tip material, wherein the first tip material comprises a polyether block amide having a Shore D durometer hardness of 50 to 60 and the second tip material comprises a polyether block amide having a Shore D durometer hardness of 60 to 70.

5. The catheter tip of claim 4, wherein the proximal segment comprises the first tip material and the distal segment comprises the second tip material.

6. The catheter tip of claim 1, wherein the at least two tip materials include a first tip material and a second tip material, wherein the first tip material has a Shore D durometer hardness of 55 and the second tip material has a Shore D durometer hardness of 63.

7. The catheter tip of claim 6, wherein the proximal segment comprises the first tip material and the distal segment comprises the second tip material.

8. The catheter tip of any preceding claim, wherein the proximal segment is formed from a first tip material (120) and the distal segment (300) is formed from a second tip material (130) and the midsection (302) is formed from both the first tip material (120) and the second tip material (130).

9. The catheter tip of claim 8 made by placing the second tip material within an inner lumen of the first tip material.

10. The catheter tip of claim 8 or 9, wherein the midsection (302) is formed from heat fusing the overlapping configuration of the first tip material (120) and the second tip material (130) to create the transition which is smooth.

11. A method of making the catheter tip of claim 1, wherein the at least two materials comprises first and second tip materials, wherein the method includes providing a mandrel (110) and a holding hypotube (140), the first tip material, the second tip material, and the holding hypotube are assembled over the mandrel in that the first tip material is placed over the mandrel and the hypotube, and the second tip material is placed over the mandrel and under the first tip material, wherein the first tip material has an outer diameter that is greater than the outer diameter of the second tip material, a shrink tube of heat-shrink material is then placed around at least a junction of the first and second tip materials, the shrink tube is heated, the first and second tip materials cooled, and the shrink tube and the hypotube are removed, thereby fusing the first and second tip materials to create the transition of the midsection.

## Patentansprüche

1. Katheterspitze (600), die ein proximales Segment (304), ein distales Segment (300) und ein mittleres Teilstück (302) zwischen denselben umfasst, wobei das mittlere Teilstück mindestens zwei Spitzenmaterialien (120, 130) in einer überlappenden Konfiguration und miteinander verschmolzen einschließt, und das distale Segment eines der mindestens zwei Spitzenmaterialien einschließt, **dadurch gekennzeichnet, dass** das mittlere Teilstück einen Steifigkeitsübergang zwischen dem proximalen Segment und dem distalen Segment bereitstellt, wobei das proximale Segment steifer ist als das distale Segment.

2. Katheterspitze nach Anspruch 1, wobei sich die Katheterspitze vom proximalen Segment zum distalen Segment verjüngt.

3. Katheterspitze nach Anspruch 1, wobei das proximale Segment so konfiguriert ist, dass es betriebsmäßig mit einem Schaft eines Katheterkörpers koppelt und sich mit einem Abschnitt des Schafts des Katheterkörpers überlappt.

4. Katheterspitze nach Anspruch 1, wobei die mindestens zwei Spitzenmaterialien ein erstes Spitzenmaterial und ein zweites Spitzenmaterial einschließen, wobei das erste Spitzenmaterial ein Polyetherblockamid umfasst, das eine Shore D-Durometerhärte von 50 bis 60 aufweist, und das zweite Spitzenmaterial ein Polyetherblockamid umfasst, das eine Shore D-Durometerhärte von 60 bis 70 aufweist.

5. Katheterspitze nach Anspruch 4, wobei das proximale Segment das erste Spitzenmaterial umfasst, und das distale Segment das zweite Spitzenmaterial umfasst.

6. Katheterspitze nach Anspruch 1, wobei die mindestens zwei Spitzenmaterialien ein erstes Spitzenmaterial und ein zweites Spitzenmaterial einschließen, wobei das erste Spitzenmaterial eine Shore D-Durometerhärte von 55 aufweist, und das zweite Spitzenmaterial eine Shore D-Durometerhärte von 63 aufweist.

7. Katheterspitze nach Anspruch 6, wobei das proximale Segment das erste Spitzenmaterial umfasst, und das distale Segment das zweite Spitzenmaterial umfasst.

8. Katheterspitze nach einem vorstehenden Anspruch, wobei das proximale Segment aus einem ersten Spitzenmaterial (120) gebildet ist, und das distale Segment (300) aus einem zweiten Spitzenmaterial (130) gebildet ist, und das mittlere Teilstück (302) aus sowohl dem ersten Spitzenmaterial (120) als auch dem zweiten Spitzenmaterial (130) gebildet ist.

9. Katheterspitze nach Anspruch 8, die durch Platzieren des zweiten Spitzenmaterials innerhalb eines Innenlumens des ersten Spitzenmaterials hergestellt wird.

10. Katheterspitze nach Anspruch 8 oder 9, wobei das mittlere Teilstück (302) aus Heißverschmelzen der überlappenden Konfiguration des ersten Spitzenmaterials (120) und des zweiten Spitzenmaterials (130) gebildet wird, um den Übergang zu schaffen, der glatt ist.

11. Verfahren zur Herstellung der Katheterspitze nach Anspruch 1, wobei die mindestens zwei Materialien erste und zweite Spitzenmaterialien umfassen, wobei das Verfahren das Bereitstellen eines Dorns (110) und eines Halte-Hyposchlauchs (140) einschließt, das erste Spitzenmaterial, das zweite Spitzenmaterial und der Halte-Hyposchlauch über dem Dorn verbunden werden, indem das erste Spitzenmaterial über dem Dorn und dem Hyposchlauch platziert wird, und das zweite Spitzenmaterial über dem Dorn und unter dem ersten Spitzenmaterial platziert wird, wobei das erste Spitzenmaterial einen Außendurchmesser aufweist, der größer ist als der Außendurchmesser des zweiten Spitzenmaterials, ein Schrumpfschlauch aus heißschrumpfbarem Material dann um mindestens einen Stoß des ersten und zweiten Spitzenmaterials herum platziert wird, der Schrumpfschlauch erhitzt wird, das erste und zweite Spitzenmaterial gekühlt und der Schrumpfschlauch und der Hyposchlauch entfernt werden, wodurch das erste und zweite Spitzenmaterial verschmolzen werden, um den Übergang des mittleren Teilstücks zu schaffen.

## Revendications

1. Pointe de cathéter (600) comprenant un segment proximal (304), un segment distal (300) et une section intermédiaire (302) entre eux, dans laquelle la section intermédiaire inclut au moins deux matériaux de pointe (120, 130) dans une configuration de chevauchement et fusionnant ensemble et le segment distal inclut l'un des au moins deux matériaux de pointe, **caractérisée en ce que** la section intermédiaire fournit une transition de rigidité entre le segment proximal et le segment distal, dans laquelle le segment proximal est plus rigide que le segment distal.

2. Pointe de cathéter selon la revendication 1, dans laquelle la pointe de cathéter s'effile depuis le segment proximal vers le segment distal.

3. Pointe de cathéter selon la revendication 1, dans laquelle le segment proximal est configuré pour s'accoupler fonctionnellement à une tige d'un corps de cathéter et pour chevaucher une partie de la tige du corps de cathéter.

4. Pointe de cathéter selon la revendication 1, dans laquelle les au moins deux matériaux de pointe incluent un premier matériau de pointe et un second matériau de pointe, dans laquelle le premier matériau de pointe comprend un polyéther bloc amide ayant une dureté au duromètre Shore D de 50 à 60 et le second matériau de pointe comprend un polyéther bloc amide ayant une dureté au duromètre Shore D de 60 à 70.

5. Pointe de cathéter selon la revendication 4, dans laquelle le segment proximal comprend le premier matériau de pointe et le segment distal comprend le second matériau de pointe.

6. Pointe de cathéter selon la revendication 1, dans laquelle les au moins deux matériaux de pointe incluent un premier matériau de pointe et un second matériau de pointe, dans laquelle le premier matériau de pointe a une dureté au duromètre Shore D de 55 et le second matériau de pointe a une dureté au duromètre Shore D de 63.

7. Pointe de cathéter selon la revendication 6, dans laquelle le segment proximal comprend le premier matériau de pointe et le segment distal comprend le second matériau de pointe.

8. Pointe de cathéter selon l'une quelconque des revendications précédentes, dans laquelle le segment proximal est formé à partir d'un premier matériau de pointe (120) et le segment distal (300) est formé à partir d'un second matériau de pointe (130) et la section intermédiaire (302) est formée à la fois à partir du premier matériau de pointe (120) et du second matériau de pointe (130).

9. Pointe de cathéter selon la revendication 8 créée en plaçant le second matériau de pointe dans une lumière intérieure du premier matériau de pointe.

10. Pointe de cathéter selon la revendication 8 ou 9, dans laquelle la section intermédiaire (302) est formée à partir de la thermofusion de la configuration de chevauchement du premier matériau de pointe (120) et du second matériau de pointe (130) pour créer la transition qui est lisse.

11. Procédé de fabrication de la pointe de cathéter selon la revendication 1, dans lequel les au moins deux matériaux comprennent des premier et second matériaux de pointe, dans lequel le procédé inclut la fourniture d'un mandrin (110) et d'un hypotube de maintien (140), le premier matériau de pointe, le second matériau de pointe et l'hypotube de maintien sont assemblés sur le mandrin en ce que le premier matériau de pointe est placé par-dessus le mandrin et l'hypotube, et le second matériau de pointe est placé par-dessus le mandrin et sous le premier matériau de pointe, dans lequel le premier matériau de pointe a un diamètre extérieur qui est supérieur au diamètre extérieur du second matériau de pointe, un tube rétrécissable de matériau thermo-rétrécissable est ensuite placé autour d'au moins une jonction des premier et second matériaux de pointe, le tube rétrécissable est chauffé, les premier et second matériaux de pointe refroidis, et le tube rétrécissable et l'hypotube sont retirés, faisant ainsi fusionner les premier et second matériaux de pointe pour créer la transition de la section intermédiaire.
